(19) **Europäisches Patentamt / European Patent Office / Office européen des brevets**

(11) **EP 3 167 914 B1**

(12) **EUROPEAN PATENT SPECIFICATION**

(45) Date of publication and mention of the grant of the patent:
**29.09.2021 Bulletin 2021/39**

(21) Application number: **15818968.8**

(22) Date of filing: **10.07.2015**

(51) Int Cl.:
**A61L 31/00** *(2006.01)* **A61M 31/00** *(2006.01)*
**A61M 35/00** *(2006.01)* **A61L 31/14** *(2006.01)*

(86) International application number:
**PCT/JP2015/069925**

(87) International publication number:
**WO 2016/006693 (14.01.2016 Gazette 2016/02)**

(54) **VISIBILITY-ENSURING MATERIAL, AND DEVICE FOR DISCHARGING VISIBILITY-ENSURING MATERIAL**

MATERIAL ZUR SICHERSTELLUNG DER SICHTBARKEIT UND VORRICHTUNG ZUM ENTLADEN DES MATERIALS ZUR SICHERSTELLUNG DER SICHTBARKEIT

MATÉRIAU ASSURANT LA VISIBILITÉ, ET DISPOSITIF D'ÉVACUATION DE MATÉRIAU ASSURANT LA VISIBILITÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **11.07.2014 JP 2014143044**

(43) Date of publication of application:
**17.05.2017 Bulletin 2017/20**

(73) Proprietor: **Menicon Co., Ltd.**
**Nagoya-shi, Aichi 460-0006 (JP)**

(72) Inventors:
• **UESUGI, Koji**
 **Kasugai-shi**
 **Aichi 487-0032 (JP)**
• **NIWA, Kazuharu**
 **Kasugai-shi**
 **Aichi 487-0032 (JP)**
• **YOKOI, Hidenori**
 **Kasugai-shi**
 **Aichi 487-0032 (JP)**
• **YOKOYAMA, Yasuhiro**
 **Kasugai-shi**
 **Aichi 487-0032 (JP)**
• **NAGAI, Yusuke**
 **Nagoya-shi**
 **Aichi 452-0805 (JP)**

• **SAKAGUCHI, Hirokazu**
 **Suita-shi**
 **Osaka 565-0871 (JP)**
• **NISHIDA, Kohji**
 **Suita-shi**
 **Osaka 565-0871 (JP)**
• **MATSUSHITA, Kenji**
 **Suita-shi**
 **Osaka 565-0871 (JP)**

(74) Representative: **Plasseraud IP**
**66, rue de la Chaussée d'Antin**
**75440 Paris Cedex 09 (FR)**

(56) References cited:
EP-A1- 2 703 014     WO-A1-2010/024209
WO-A1-2013/142374     WO-A1-2014/089472
WO-A1-2015/098515     JP-A- H0 994 402
JP-A- H11 512 778     JP-A- S60 501 193
JP-A- 2008 539 257     US-A- 6 031 017

• 'Rapidly in situ forming chitosan/epsilon-polylysine hydrogels for adhesive sealants and hemostatic materials' CARBOHYDR POLYM vol. 96, no. 1, July 2013, pages 342 - 348, XP028544311
• None

SEQUENCE LISTING

<110> Menicon Co. Ltd.

<120> Materials for ensuring visibility and discharging equipment for the materials for ensuring visibility

<130> MNC14037PCT

<150> JP2014-143044
<151> 2014-07-11

<160> 19

<170> PatentIn version 3.5

<210> 1

<211> 13
<212> PRT
<213> Artificial Sequence

<220>
<223> a self-assembling peptide that can be used in the present invention

<400> 1

```
Arg Leu Asp Leu Arg Leu Ala Leu Arg Leu Asp Leu Arg
1               5                   10
```

<210> 2
<211> 13
<212> PRT
<213> Artificial Sequence

<220>
<223> a self-assembling peptide that can be used in the present invention

<400> 2

```
Arg Leu Asp Leu Arg Leu Leu Leu Arg Leu Asp Leu Arg
1               5                   10
```

<210> 3
<211> 13
<212> PRT
<213> Artificial Sequence

<220>
<223> a self-assembling peptide that can be used in the present invention

<400> 3

```
Arg Ala Asp Leu Arg Leu Ala Leu Arg Leu Asp Leu Arg
1               5                   10
```

<210> 4
<211> 13
<212> PRT
<213> Artificial Sequence

<220>
<223> a self-assembling peptide that can be used in the present invention

<400> 4

```
Arg Leu Asp Leu Arg Leu Ala Leu Arg Leu Asp Ala Arg
1               5                   10
```

<210> 5
<211> 13
<212> PRT
<213> Artificial Sequence

<220>
<223> a self-assembling peptide that can be used in the present invention

<400> 5

```
Arg Ala Asp Leu Arg Leu Leu Leu Arg Leu Asp Leu Arg
1               5                   10
```

<210> 6
<211> 13
<212> PRT
<213> Artificial Sequence

<220>
<223> a self-assembling peptide that can be used in the present invention

<400> 6

```
Arg Ala Asp Leu Arg Leu Leu Leu Arg Leu Asp Ala Arg
1               5                   10
```

<210> 7
<211> 13
<212> PRT
<213> Artificial Sequence

<220>
<223> a self-assembling peptide that can be used in the present invention

<400> 7

```
Arg Leu Asp Leu Arg Ala Leu Leu Arg Leu Asp Leu Arg
1               5                   10
```

<210> 8
<211> 13
<212> PRT
<213> Artificial Sequence

<220>
<223> a self-assembling peptide that can be used in the present invention

<400> 8

```
Arg Leu Asp Leu Arg Leu Leu Ala Arg Leu Asp Leu Arg
1               5                   10
```

<210> 9
<211> 16
<212> PRT
<213> Artificial Sequence

<220>
<223> a self-assembling peptide that can be used in the present invention

<400> 9

```
Arg Ala Ser Ala Arg Ala Asp Ala Arg Ala Ser Ala Arg Ala Asp Ala
1               5               10                  15
```

<210> 10

<211> 16
<212> PRT
<213> Artificial Sequence

<220>
<223> a self-assembling peptide that can be used in the present invention

<400> 10

```
    Arg Ala Asn Ala Arg Ala Asp Ala Arg Ala Asn Ala Arg Ala Asp Ala
    1               5                   10                  15
```

<210> 11
<211> 16
<212> PRT
<213> Artificial Sequence

<220>
<223> a self-assembling peptide that can be used in the present invention

<400> 11

```
    Arg Ala Ala Ala Arg Ala Asp Ala Arg Ala Ala Ala Arg Ala Asp Ala
    1               5                   10                  15
```

<210> 12
<211> 16
<212> PRT
<213> Artificial Sequence

<220>
<223> a self-assembling peptide that can be used in the present invention

<400> 12

```
    Arg Ala Ser Ala Arg Ala Asp Ala Arg Ala Asp Ala Arg Ala Ser Ala
    1               5                   10                  15
```

<210> 13
<211> 16
<212> PRT
<213> Artificial Sequence

<220>
<223> a self-assembling peptide that can be used in the present invention

<400> 13

```
    Arg Ala Asp Ala Arg Ala Ser Ala Arg Ala Ser Ala Arg Ala Asp Ala
    1               5                   10                  15
```

<210> 14
<211> 16
<212> PRT
<213> Artificial Sequence

<220>
<223> a self-assembling peptide that can be used in the present invention

<400> 14

```
        Arg Ala Ser Ala Arg Ala Ser Ala Arg Ala Ser Ala Arg Ala Asp Ala
        1               5                   10                  15
```

<210> 15
<211> 12
<212> PRT
<213> Artificial Sequence

<220>
<223> a self-assembling peptide that can be used in the present invention

<400> 15

```
                Arg Ala Ser Ala Arg Ala Asp Ala Arg Ala Ser Ala
                1               5                   10
```

<210> 16
<211> 16
<212> PRT
<213> Artificial Sequence

<220>
<223> a self-assembling peptide that can be used in the present invention

<400> 16

```
        Lys Ala Ser Ala Lys Ala Glu Ala Lys Ala Ser Ala Lys Ala Glu Ala
        1               5                   10                  15
```

<210> 17
<211> 16
<212> PRT
<213> Artificial Sequence

<220>
<223> a self-assembling peptide that can be used in the present invention

<400> 17

```
        Ser Ala Glu Ala Lys Ala Glu Ala Ser Ala Glu Ala Lys Ala Glu Ala
        1               5                   10                  15
```

<210> 18
<211> 12
<212> PRT
<213> Artificial Sequence

<220>
<223> a self-assembling peptide that can be used in the present invention

<400> 18

```
                Lys Leu Ser Leu Lys Leu Asp Leu Lys Leu Ser Leu
                1               5                   10
```

<210> 19

<211> 12
<212> PRT
<213> Artificial Sequence

<220>
<223> a self-assembling peptide that can be used in the present invention

<400> 19

Lys Leu Ala Leu Lys Leu Asp Leu Lys Leu Ala Leu
1                   5                   10

## Description

### Technical Field

The present invention relates to a device to be used for discharging a visibility-ensuring material.

### Background Art

As means for ensuring, during surgery, a surgical field from bleeding or the like, gauze, saline, an electric scalpel, fibrin glue, and the like are used. When gauze and saline are used, it is necessary to remove body fluid constantly, and the time period, during which a surgical field is ensured, is short. When an electric scalpel is used, a bleeding point cannot be found in case of heavy bleeding, and there is a risk in that an unnecessary portion may be burnt. There is also a risk in that an operative scar may remain in a tissue. Fibrin glue sticks to a tissue and cannot be removed easily, and hence may cause synechia. This also hinders the transmission of light. Therefore, sufficient visibility may not be ensured, and it is difficult to use fibrin glue particularly in the field of ophthalmology.

The usefulness of a gel-like viscoelastic body having transparency as a visibility-ensuring material is drawing attention. For example, a self-assembling peptide is capable of self-assembling in an aqueous solution to form a gel having thixotropic characteristics (hereinafter referred to as "self-assembling peptide gel") (for example, Patent Literatures 1 and 2). In recent years, such gel forming ability has been paid attention to, and there has been proposed that the self-assembling peptide be used for medical applications, e.g., a hemostatic material (for example, Patent Literatures 3 and 4). However, in order to ensure visibility required for performing delicate operations, such as dissection and suturation, through a gel, the self-assembling peptide is susceptible to improvement.

WO 99/17821 discloses a device for introducing a viscoelastic substance into the intraocular space of a patient's eye, the device comprising a syringe with a main body portion and a needle portion and a composition comprising the viscoelastic substance within the main body of the syringe. However, WO 99/17821 does not teach that the viscoelastic substance can be a self-assembling peptide used as a visibility-ensuring material.

### Citation List

### Patent Literature

[PTL 1] US 5670483 A
[PTL 2] WO 2010/103887 A1
[PTL 3] JP 5204646 B2
[PTL 4] WO 2006/116524 A1

### Summary of Invention

### Technical Problem

The inventors of the present invention have found that the physical characteristics (for example, strength) of the gel-like viscoelastic body influence its prevention effect on inflow of a body fluid, such as blood, into a surgical field. However, the inventors of the present invention have found that, when the gel-like viscoelastic body having strength capable of satisfactorily exhibiting a prevention effect on inflow of a body fluid is applied to a wound area from a syringe, the gel is formed into a string shape and becomes inferior in covering property of the wound area in some cases . The gel discharged, as it is, has many irregularities on the surface, and hence the visibility of the surgical field may become insufficient even when the gel is transparent.

The present invention has been made to solve the above-mentioned problems. Is described herein a visibility-ensuring material which is capable of ensuring a surgical field for a predetermined time period, which does not inhibit the transmission of light, and which is capable of ensuring the visibility with which even delicate operations can be satisfactorily performed.

### Solution to Problem

A visibility-ensuring material described herein includes a gel-like viscoelastic body. The visibility-ensuring material has a storage elastic modulus during use of from 40 Pa to 700 Pa.

The visibility-ensuring material may have an area measured by spreading test evaluation of 2.0 cm$^2$ or more.

The visibility-ensuring material described herein is used for treatment in digestive surgery, dermatology, urology, gynecology, ophthalmology, neurosurgery, otolaryngology, cardiovascular surgery, orthopedic surgery, plastic surgery, oral surgery, and dentistry.

In particular, the visibility-ensuring material is used for treatment in ophthalmology.

The present invention provides a discharge device for a visibility-ensuring material comprising: a syringe, wherein a gel-like viscoelastic body is filled into the syringe; and at least one of: a mesh filter having an opening of less than 1 mm, a needle having an inner diameter of less than 1 mm, and an application nozzle having a tip end having an inner diameter of less than 1 mm, wherein the gel-like viscoelastic body comprises a self-assembling peptide, and wherein the gel-like viscoelastic body is intended to be passed through the mesh filter, the needle and/or the application nozzle.

In one embodiment, the mesh filter has a mesh size of more than 10-mesh.

In one embodiment, the sum of charges of amino acid residues constituting the self-assembling peptide at pH 7.0 is from -3 to -1 or from +1 to +3.

In one embodiment, the self-assembling peptide comprises a peptide formed of an amino acid sequence selected from SEQ ID Nos: 1 to 19.

Advantageous Effects of Invention

A visibility-ensuring material which prevents a body fluid, such as blood, from entering a surgical field and which is capable of ensuring the visibility with which even delicate operations can be satisfactorily performed is described herein. The described visibility-ensuring material rapidly spreads onto a tissue to which the visibility-ensuring material is applied and is also capable of preventing troubles such as running off from the tissue. After being used, the visibility-ensuring material can be easily removed from the tissue, to thereby prevent the adverse effects of the use of the visibility-ensuring material on the tissue.

Brief Description of Drawings

FIGS. **1** are each a schematic perspective view for illustrating an exploded state of a discharge device according to one embodiment of the present invention. FIG. **1(a)** is an illustration of an outer casing, FIG. **1(b)** is an illustration of a plunger, and FIG. **1(c)** is an illustration of a filter unit.

FIG. **2** is a schematic perspective view for illustrating an assembled state of the discharge device illustrated in FIGS. **1.**

FIG. **3** is a schematic sectional view of a filter unit.

FIG. **4** is a schematic perspective view for illustrating an assembled state of a discharge device according to another embodiment of the present invention.

FIG. **5A is** a schematic perspective view for illustrating an example of an application nozzle capable of being mounted as a part of the discharge device of the present invention.

FIG. **5B** is a schematic perspective view for illustrating a state in which the application nozzle is mounted on the discharge device of FIG. **2.**

FIG. **5C** is a schematic perspective view for illustrating a state in which the application nozzle is mounted on the discharge device of FIG. **4.**

FIG. **6(a)** is a photograph of a self-assembling peptide gel immediately after being discharged in Example 4, and FIG. **6(b)** is a photograph of a self-assembling peptide gel immediately after being discharged in Comparative Example 1.

FIG. **7** is a graph for showing a storage elastic modulus of a self-assembling peptide gel.

Description of Embodiments

[A. Visibility-ensuring Material]

A visibility-ensuring material described herein contains a gel-like viscoelastic body. The term "gel" as used herein means a viscoelastic substance having both a viscous property and an elastic property. Specifically, the gel may refer to a substance in which "G'>G''" is satisfied when a storage elastic modulus G' and a loss elastic modulus G'' are measured by dynamic viscoelastic measurement.

The storage elastic modulus during use (for example, during contact with a tissue to which the visibility-ensuring material is applied) of the visibility-ensuring material is from 40 Pa to 700 Pa, preferably from 100 Pa to 600 Pa, more preferably from 150 Pa to 500 Pa. When the storage elastic modulus falls within the above-mentioned range, a body fluid, such as blood, is prevented from entering a surgical field, and satisfactory visibility can be ensured. Even when a discharge device for a visibility-ensuring material including a syringe is used as a discharge device for a visibility-ensuring material described later, the visibility-ensuring material can be discharged more easily. The term "syringe" as used herein refers to a syringe including an outer casing and a plunger. The term "storage elastic modulus" as used herein means, unless otherwise stated, a storage elastic modulus (G') at a time when the angular frequency is 1 radian/second in dynamic viscoelastic measurement that is performed while a frequency is changed at 37°C through use of a rotary rheometer.

The storage elastic modulus during non-use of the visibility-ensuring material may fall outside the above-mentioned range. For example, when a visibility-ensuring material having a storage elastic modulus of more than 700 Pa is used while being discharged through use of a discharge device described later, the storage elastic modulus during use is allowed to fall within the above-mentioned range.

It is preferred that the visibility-ensuring material described herein have appropriate ease of spreading. As an indicator of the ease of spreading, there is given, for example, an area in the following spreading test. The area of the visibility-ensuring material in this spreading test is preferably 2.0 cm$^2$ or more, more preferably 2.5 cm$^2$ or more, still more preferably 3.0 cm$^2$ or more. The visibility-ensuring material having such characteristic can rapidly spread onto a tissue to which the visibility-ensuring material is applied, to thereby cover a desired portion. Even when the desired portion extends over a wide range, the surgical field can be sufficiently covered to ensure visibility without use of a large amount of the visibility-ensuring material. It is preferred that the area be, for example, 6 cm$^2$ or less. As a method of evaluating the ease of spreading in the spreading test, there is given a method described in Examples.

The visible light transmittance of the visibility-ensuring material is preferably 80% or more, more preferably 85% or more, still more preferably 90% or more. When the visible light transmittance of the visibility-ensuring material falls within the above-mentioned range, the surgical field can be satisfactorily ensured. The term "visible light transmittance" as used herein refers to a transmittance of light having a wavelength of from 380 nm to 780 nm and may be measured through use of, for example, a spectrophotometer.

The visibility-ensuring material described herein has a pH of preferably from 4 to 9, more preferably from 5 to 8, still more preferably from 5.5 to 7.5, particularly preferably from 6 to 7. When the pH falls within the above-mentioned range, cytotoxicity can be reduced. When a self-assembling peptide is used as the viscoelastic body, the hydrolysis of the self-assembling peptide during heating can be avoided. Therefore, the visibility-ensuring material can be subjected to sterilization treatment involving heating, such as high-pressure steam sterilization. Further, an originally intended intermolecular interaction can be allowed to occur between the self-assembling peptides.

The visibility-ensuring material described herein has an osmotic pressure of preferably from 200 mOsm/kg·$H_2O$ to 400 mOsm/kg·$H_2O$, more preferably from 257 mOsm/kg·$H_2O$ to 372 mOsm/kg·$H_2O$. When the osmotic pressure falls within the above-mentioned range, cytotoxicity can be reduced.

[A-1. Gel-like Viscoelastic Body]

As the gel-like viscoelastic body, any appropriate viscoelastic body may be used depending on the purpose and the like. Specific examples thereof include: a self-assembling peptide gel; polysaccharide gels, such as hyaluronic acid, carrageenan, and sacran; a metal colloid solution; grease; and solutions of polymers, such as polyacrylic acid and cellulose. Of those, a self-assembling peptide gel is preferred because the effect is suitably obtained. Through use of the self-assembling peptide gel, the viscoelastic body has high adhesiveness to a tissue and can easily cover a desired portion and a surrounding portion thereof.

It is also preferred that the viscoelastic body have thixotropic characteristics. With such characteristics, the viscoelastic body can spontaneously recover strength (storage elastic modulus) after application and exhibit an effect of suppressing the intrusion of a body fluid into the surgical field. Further, the gel surface becomes smooth without irregularities, and hence the visibility of an affected area is enhanced, and the surgical field can be easily confirmed.

As the self-assembling peptide gel, any appropriate gel formed by self-assembling of a self-assembling peptide may be used. Typically, the self-assembling peptide gel is formed from an aqueous solution containing a self-assembling peptide.

[A-1-1. Self-assembling Peptide]

As the self-assembling peptide, there may be used any appropriate peptide capable of spontaneously assembling in an aqueous solution to form a gel through an interaction of peptide molecules. More specifically, there maybe preferably used a peptide capable of spontaneously assembling in an aqueous solution to form fibrous molecular assemblies through an interaction of peptide molecules and developing a three-dimensional network structure to form a gel through an interaction of the molecular assemblies. As the interaction of the peptide molecules, there are given, for example: an electrostatic interaction, such as hydrogen bonding, interionic interaction, or van der Waals force; and a hydrophobic interaction. Further, the formation of the fibrous molecular assemblies may be confirmed by microscopic observation.

The amino acids constituting the self-assembling peptide may be L-amino acids or D-amino acids. The amino acids are preferably L-amino acids. In addition, the amino acids maybe natural amino acids or non-natural amino acids. The amino acids are preferably natural amino acids because the natural amino acids are available at low cost and peptides can be easily synthesized therefrom.

The sum of charges of amino acid residues constituting the self-assembling peptide at pH 7.0 is preferably from -3 to -1 or from +1 to +3, more preferably -3, -2, +2, or +3. When positive charges and negative charges derived from side chains of amino acid residues included in the self-assembling peptide in a neutral region are not balanced as mentioned above, static attraction and repulsion suitable for formation of a gel are balanced, resulting in forming a transparent and stable gel in a neutral region. The term "neutral region" as used herein refers to a region having a pH of from 6.0 to 8.5, preferably from 6.5 to 8.0, more preferably 7.0.

The charges of the self-assembling peptide at different pH values may be determined, for example, by a program available on a website of PROTEIN CALCULATOR v3.4 (http://protcalc.sourceforge.net/).

As a specific example of the self-assembling peptide that is preferably used, there is given a peptide having an amino acid sequence represented by the following formula (I):

$$a_1 b_1 c_1 b_2 a_2 b_3 d b_4 a_3 b_5 c_2 b_6 a_4 \quad (I)$$

where: $a_1$ to $a_4$ each represent a basic amino acid residue; $b_1$ to $b_6$ each represent a non-charged polar amino acid residue and/or a hydrophobic amino acid residue, provided that at least five thereof each represent a hydrophobic amino acid residue; $c_1$ and $c_2$ each represent an acidic amino acid residue; and d represents a hydrophobic amino acid residue.

In the amino acid sequence, $a_1$ to $a_4$ each represent a basic amino acid residue . The basic amino acid is preferably arginine, lysine, or histidine, more preferably arginine or lysine because those amino acids are highly basic. $a_1$ to $a_4$ may represent the same amino acid residue or different amino acid residues.

In the amino acid sequence, $b_1$ to $b_6$ each represent a non-charged polar amino acid residue and/or a hydrophobic amino acid residue, provided that at least five thereof each represent a hydrophobic amino acid residue. The hydrophobic amino acid is preferably alanine, leucine, isoleucine, valine, methionine, phenylalanine, tryptophan, glycine, or proline. The non-charged polar amino acid is preferably tyrosine, serine, threonine, asparagine, glutamine, or cysteine, because those amino acids are easily available.

It is preferred that $b_3$ and $b_4$ each independently represent any appropriate hydrophobic amino acid residue. It is more preferred that $b_3$ and $b_4$ each independently represent a leucine residue, an alanine residue, a valine residue, or an isoleucine residue. It is particularly preferred that $b_3$ and $b_4$ each independently represent a leucine residue or an alanine residue.

It is preferred that all of $b_1$ to $b_6$ represent hydrophobic amino acid residues. This is because the self-assembling peptide can suitably form a $\beta$-sheet structure to self-assemble. It is more preferred that $b_1$ to $b_6$ each independently represent a leucine residue, an alanine residue, a valine residue, or an isoleucine residue. It is still more preferred that $b_1$ to $b_6$ each independently represent a leucine residue or an alanine residue. Preferably, four or more of $b_1$ to $b_6$ represent leucine residues, it is more preferred that five or more of $b_1$ to $b_6$ represent leucine residues, and it is still more preferred that all of $b_1$ to $b_6$ represent leucine residues.

In the amino acid sequence, $c_1$ and $c_2$ each represent an acidic amino acid residue. The acidic amino acid is preferably aspartic acid or glutamic acid, because those amino acids are easily available. $c_1$ and $c_2$ may represent the same amino acid residue or different amino acid residues.

In the amino acid sequence, d represents a hydrophobic amino acid residue. d preferably represents an alanine residue, a valine residue, a leucine residue, or an isoleucine residue.

Preferably, two of three continuous amino acid residues, $b_3$, d, and $b_4$, represent leucine residues and the remainder represents an alanine residue. In this case, any of $b_3$, d, and $b_4$ may represent an alanine residue. In addition, preferably, all of the three continuous amino acid residues, $b_3$, d, and $b_4$, represent leucine residues.

Preferred specific examples of the amino acid sequence represented by the formula (I) are listed below.

n-RLDLRLALRLDLR-c (SEQ ID NO: 1)
n-RLDLRLLLRLDLR-c (SEQ ID NO: 2)
n-RADLRLALRLDLR-c (SEQ ID NO: 3)
n-RLDLRLALRLDAR-c (SEQ ID NO: 4)
n-RADLRLLLRLDLR-c (SEQ ID NO: 5)
n-RADLRLLLRLDAR-c (SEQ ID NO: 6)
n-RLDLRALLRLDLR-c (SEQ ID NO: 7)
n-RLDLRLLARLDLR-c (SEQ ID NO: 8)

As another self-assembling peptide that may be preferably used, there is given a peptide described in WO 2007/000979 A1, i.e., a self-assembling peptide including polar amino acid residues and non-polar amino acid residues (hydrophobic amino acid residues), in which the self-assembling peptide includes an acidic amino acid residue and a basic amino acid residue as the polar amino acid residues, a sum of charge of the acidic amino acid residue and charge of the basic amino acid residue in a neutral region is a number excluding 0, and the self-assembling peptide is capable of forming a β-sheet structure in which only the non-polar amino acid residues are arranged on one surface upon self-assembly in an aqueous solution.

Of the self-assembling peptides, a peptide including an acidic amino acid residue, a basic amino acid residue, and a non-charged polar amino acid as polar amino acids is preferred. Preferred specific examples of such self-assembling peptide are listed below.

n-RASARADARASARADA-c (SEQ ID NO: 9)
n-RANARADARANARADA-c (SEQ ID NO: 10)
n-RAAARADARAAARADA-c (SEQ ID NO: 11)
n-RASARADARADARASA-c (SEQ ID NO: 12)
n-RADARASARASARADA-c (SEQ ID NO: 13)
n-RASARASARASARADA-c (SEQ ID NO: 14)
n-RASARADARASA-c (SEQ ID NO: 15)
n-KASAKAEAKASAKAEA-c (SEQ ID NO: 16)
n-SAEAKAEASAEAKAEA-c (SEQ ID NO: 17)
n-KLSLKLDLKLSL-c (SEQ ID NO: 18)
n-KLALKLDLKLAL-c (SEQ ID NO: 19)

In addition, a specific example of the self-assembling peptide is a peptide disclosed in WO 2010/103887 A1 or US 5670483 A.

The self-assembling peptide may be produced by any appropriate production method. Examples thereof include a chemical synthetic method, such as a solid-phase method, for example, an Fmoc method or a liquid-phase method, and a molecular biological method, such as gene recombinant expression. The self-assembling peptide may be formed into any appropriate salt during purification. However, a self-assembling peptide in the form of a salt may also be used.

The self-assembling peptide may be subjected to any appropriate modification depending on a purpose or the like. A site at which the modification is performed is not particularly limited, and examples thereof include an N-terminal amino group and a C-terminal carboxyl group of a self-assembling peptide, and both of the groups.

Any appropriate modification may be selected as the modification as long as the peptide after the modification has a self-assembling ability. Examples thereof include: the introduction of a protective group, such as acetylation of the N-terminal amino group or amidation of the C-terminal carboxyl group; the introduction of a functional group, such as alkylation, esterification, or halogenation; hydrogenation; the introduction of a saccharide compound, such as a monosaccharide, a disaccharide, an oligosaccharide, or a polysaccharide; the introduction of a lipid compound, such as a fatty acid, a phospholipid, or a glycolipid; the introduction of an amino acid or a protein; the introduction of DNA; and the introduction of, for example, other compounds each having bioactivity. Only one kind of modification may be performed, or two or more kinds thereof may be performed in combination. For example, the following may be adopted: a desired amino acid is introduced into the C-terminus of the self-assembling peptide to yield an added peptide, the N-terminus of the added peptide is acetylated, and the C-terminus thereof is amidated.

When an amino acid or a protein is introduced, the number of the amino acids to be introduced is preferably from 1 to 180, more preferably from 1 to 50, still more preferably from 1 to 30, particularly preferably from 1 to 10, most preferably from 1 to 5. When the number of the amino acid residues to be introduced exceeds 180, the self-assembling ability may be impaired.

The concentration of the self-assembling peptide in the visibility-ensuring material described herein may be appropriately set in accordance with a desired storage elastic modulus or the like. The concentration of the self-assembling peptide is preferably from 0.5 wt% to 3.0 wt%, more preferably from 0.8 wt% to 2.8 wt%, still more preferably from 1.1 wt% to 2.5 wt%, yet still more preferably from 1.15 wt% to 2.0 wt%, even yet still more preferably from 1.2 wt% to 1.8 wt%.

[A-1-2. Water]

As water, purified water, such as ion-exchanged water or distilled water, may be preferably used.

The water content ratio (%) of the viscoelastic body (=weight of water in viscoelastic body/total weight of viscoelastic body×100) is, for example, from 85% to 99.9%, preferably from 86% to 99%, more preferably from 87% to 95%.

[A-1-3. Viscoelastic Body other than Self-assembling Peptide Gel]

As described above, a gel-like viscoelastic body having a storage elastic modulus during use of from 40 Pa to 700 Pa is used. A viscoelastic body that may be used except for the self-assembling peptide gel may be prepared through use of, for example, any appropriate thickener, gelling agent, and the like, and water. As the thickener and the gelling agent, those which are commercially available may be used. Specifically, there are given a polyacrylic acid-based polymer, e.g., "Rheogic 270" (trade name) manufactured by Toagosei Co., Ltd., a cellulose-based polymer, e.g., "Sangelose 90L" (trade name) manufactured by Daido Chemical Industry Co., Ltd., and the like. Even when a viscoelastic body other than the self-assembling peptide is used, it is only necessary that the water content ratio of the water and the viscoelastic body fall within the above-mentioned range.

[A-2. Additive]

The visibility-ensuring material described herein may further contain any appropriate additive as necessary. For example, the visibility-ensuring material preferably contains a pH adjuster.

As the pH adjuster, there are given: acids, such as hydrochloric acid, citric acid, and acetic acid; bases, such as sodium hydroxide and potassium hydroxide; salts of weak acids and strong bases, such as sodium hydrogen carbonate and sodium carbonate; histidine; and the like.

The addition amount of the pH adjuster may be appropriately set in accordance with a desired pH.

As other additives that may be contained in the visibility-ensuring material described herein, there are given, for example: a buffer; a tonicity agent; amino acids; vitamins; alcohols; drugs; and the like. Those other additives may be used alone or two or more kinds thereof may be used in combination.

Examples of the buffer include: phosphoric acid and phosphates, such as sodium phosphate, sodium dihydrogen phosphate, disodium hydrogen phosphate, potassium phosphate, potassium dihydrogen phosphate, and dipotassium hydrogen phosphate; boric acid and borates, such as borax, sodium borate, and potassium borate; citrates, such as sodium citrate and disodium citrate; acetates, such as sodium acetate and potassium acetate; Tris; and HEPES.

Examples of the tonicity agent include: chlorides, such as sodium chloride, potassium chloride, calcium chloride, and magnesium chloride; monosaccharides, such as glucose, fructose, and galactose; disaccharides, such as sucrose, trehalose, maltose, and lactose; and sugar alcohols, such as mannitol and sorbitol.

The storage elastic modulus of the visibility-ensuring material described herein may be adjusted to from 40 Pa to 700 Pa in a stage of preparing the visibility-ensuring material. Alternatively, the storage elastic modulus of a visibility-ensuring material having a storage elastic modulus outside of the above-mentioned range may be decreased by any appropriate method so as to be adjusted to from 40 Pa to 700 Pa during use. As a method of decreasing the storage elastic modulus, there is given, for example, a method involving decreasing the storage elastic modulus by applying an external load to the gel-like viscoelastic body, and simultaneously discharging the viscoelastic body from a container. A viscoelastic body having increased flowability can be discharged through application of an external load during discharge . As a result, the adhesiveness of the gel to an application surface is enhanced, and the application effect of the gel can be enhanced. The gel surface becomes smooth, and the visibility of the application surface can also be enhanced. Further, when a gel-like viscoelastic body having thixotropic characteristics is used as the gel-like viscoelastic body to be used, the gel-like viscoelastic body recovers the original storage elastic modulus, for example, by being allowed to stand still after being discharged and can further exhibit an effect of suppressing the intrusion of a body fluid and the like.

When the storage elastic modulus is decreased by the above-mentioned method, the storage elastic modulus of the gel-like viscoelastic body before application of an external load is, for example, from 350 Pa to 3,000 Pa, more preferably from 400 Pa to 2,500 Pa. With such storage elastic modulus, the effect of suppressing the intrusion of a body fluid and the like can be further exhibited.

The decrease ratio of the storage elastic modulus ((storage elastic modulus of gel-like viscoelastic body before application of external load - storage elastic modulus of viscoelastic body immediately after being discharged)/storage elastic modulus of gel-like viscoelastic body before application of external load $\times$ 100) is preferably 5% or more, more preferably 10% or more. When the storage elastic modulus is decreased by 5% or more, the adhesiveness and covering property with respect to the application surface can be enhanced.

The external load may be any appropriate load as long as the load can apply a shearing stress to the gel-like viscoelastic body to decrease the storage elastic modulus thereof. As specific examples of the external load, there are given: applying an ultrasonic wave to the viscoelastic body; causing the viscoelastic body to pass through a needle; causing the viscoelastic body to pass through a filter; causing the viscoelastic body to pass through an application nozzle; spraying the viscoelastic body from a spray; shaking or stirring the viscoelastic body; and the like. From the viewpoints of the workability and the control of the viscoelastic body after being discharged, it is preferred that the viscoelastic body be caused to pass through the filter or sprayed with a spray. When a small amount of application is locally performed, the passage through the needle (typically, a thin needle) or the passage through the application nozzle may be preferably used.

The needle is typically a thin needle as described above . More specifically, the needle has an inner diameter of preferably 0.85 mm (18 G) or less, more preferably from 0.04 mm to 0.44 mm (from 33 G to 22 G), still more preferably from 0.05 mm to 0.38 mm (from 32 G to 23 G). When the inner diameter is too large, a sufficient load is not applied to the gel-like viscoelastic body, and the storage elastic modulus may not be decreased sufficiently. When the inner diameter is too small, an extremely large extrusion pressure is required, and the workability may be degraded.

The filter has an effective filtration area of preferably 0.1 cm$^2$ or more, more preferably from 0.5 cm$^2$ to 10 cm$^2$, still more preferably from 0.7 cm$^2$ to 5 cm$^2$. With such effective filtration area, the workability can be excellent.

The filter has an opening ratio of preferably 2% or more, more preferably from 3% to 60%, still more preferably from 3% to 50%. With such opening ratio, the workability can be excellent.

There is no particular limitation on the kind of the filter. For example, a mesh filter, a nonwoven fabric filter, a porous film filter, a punching filter, or the like may be used. Alternatively, the filter may be a layer filled with fine particles . From the viewpoints of the workability and the uniformity of the viscoelastic body after being discharged, the mesh filter is preferred.

A material for forming the filter may be appropriately selected in accordance with the kind of the viscoelastic body and the like. As the material for forming the filter, for example, a polymer, such as polyester, polyamide, or polypropylene, a metal, glass, or the like may be used.

As a method of weaving the mesh filter, there are given plain weave, twill weave, plain dutch weave, twilled dutch weave, and the like. Of those, plain weave may be preferably used.

The mesh size of the mesh filter is preferably more than 10-mesh, more preferably 50-mesh or more, still more preferably from 70-mesh to 800-mesh, yet still more preferably from 100-mesh to 600-mesh. Through use of a thin mesh filter having a mesh size of more than 10-mesh, the storage elastic modulus of the viscoelastic body can be suitably decreased. Meanwhile, through use of a mesh filter having a mesh size of 800-mesh or less, the viscoelastic body can be discharged with satisfactory workability. The unit "mesh" means the number of stitches present between a vertical line of 25.4 mm (1 inch) and a horizontal line of 25.4 mm (1 inch).

The wire diameter of the mesh filter is preferably from 10 $\mu$m to 500 $\mu$m, more preferably from 30 $\mu$m to 150 $\mu$m. With such wire diameter, appropriate strength and an efficient opening ratio can be obtained.

The opening of the mesh filter is preferably less than 1 mm, more preferably 200 $\mu$m or less, still more preferably from 3 $\mu$m to 50 $\mu$m, yet still more preferably from 5 $\mu$m to 20 $\mu$m. Through use of the mesh filter having an opening of less than 1 mm, the storage elastic modulus of the viscoelastic body can be suitably decreased. Meanwhile, through use of the mesh filter having an opening of 3 $\mu$m or more, the viscoelastic body can be discharged with satisfactory workability.

The tip end portion of the application nozzle may have any appropriate shape. Examples of the shape include a circular shape, an oval shape, a square shape, and a rectangular shape. The shape of the tip end portion may be obtained by flattening any of those shapes.

It is preferred that the size of the tip end portion of the application nozzle be as small as possible. Through use of such application nozzle, the storage elastic modulus of the viscoelastic body can be suitably decreased. For example, the size of the tip end portion is less than 1 mm. The size of the tip end portion refers to an inner diameter, for example, in the case where the tip end portion has a circular shape, and refers to a length of a short axis, for example, in the case where the tip end portion has an oval shape.

It is preferred that a two-fluid nozzle be used as the spray. The two-fluid nozzle refers to a spray nozzle involving mixing a compressed gas and a liquid to be jetted, which are separated into two systems, and jetting the mixture. The two-fluid nozzle can pulverize and atomize the liquid with a high-speed stream of the compressed gas and spray a fine mist under low pressure. The two-fluid nozzle can also jet a high-viscosity liquid, such as grease, and a viscoelastic body, such as a gel. As the gas to be used, there are given carbon dioxide, nitrogen, air, and the like. The pressure of the gas falls within a range of preferably from 0.01 MPa to 0.3 MPa, more preferably from 0.05 MPa to 0.15 MPa. When the viscoelastic body is discharged while the gas is flowed under a pressure within this range, the viscoelastic body is finely sheared to be discharged in the form of a mist. The viscoelastic body is not sufficiently sheared under a pressure of 0.01 MPa or less and may not be uniformly jetted. Under a pressure of more than 0.3 MPa, the viscoelastic body jetted onto an application surface is pushed away with the force of the gas, and the workability may be degraded.

As a container in which the gel-like viscoelastic body is to be accommodated, there may be used a container including an accommodating part configured to accommodate the viscoelastic body and a discharge port part, in which the accommodating part communicates to the outside through the discharge port part. With such a container, the viscoelastic body accommodated in the accommodating part can be discharged through the discharge port part through application of a pressure to the viscoelastic body. The opening area of the discharge port part may be, for example, 8 $mm^2$ or less, preferably from 0.03 $mm^2$ to 1 $mm^2$. It is preferred that the container further include extrusion means for excluding the viscoelastic body from the accommodating part to the outside of the discharge port part. In the present invention, the container is a syringe-type container.

The time period from the application of an external load to the viscoelastic body to the discharge of the viscoelastic body may be any appropriate time period as long as the effect of the present invention is obtained. Specifically, the viscoelastic body may be discharged from the container after a lapse of a predetermined time period (for example, from tens of seconds to several minutes) from the application of an external load. Alternatively, the viscoelastic body may be discharged from the container continuously from the application of an external load. As a specific example of the embodiment in which the viscoelastic body is discharged after a lapse of a predetermined time period from an external load, there is given an embodiment in which the storage elastic modulus is decreased by applying an ultrasonic wave to the viscoelastic body in the container, and the viscoelastic body is discharged from the container after a lapse of a predetermined time period. Meanwhile, as a specific example of the embodiment in which the viscoelastic body is discharged continuously from an external load, there is given an embodiment in which the viscoelastic body having passed through a needle, a filter, an application nozzle, or a spray unit is directly discharged.

[B. Method of manufacturing Visibility-ensuring Material]

A method of manufacturing a visibility-ensuring material described herein is obtained by, for example, mixing a gel-like viscoelastic body and any appropriate additive with each other. Specifically, when a self-assembling peptide gel is used as the viscoelastic body, the method includes mixing a self-assembling peptide, water, and any appropriate additive with each other, to thereby provide a self-assembling peptide aqueous solution (mixing step) and allowing the obtained aqueous solution to stand still to cause the self-assembling peptide to self-assemble, to thereby provide a self-assembling peptide gel (gelling step). As necessary, the method may further include, after the mixing step and before the gelling step, defoaming and/or deaerating the self-assembling peptide aqueous solution (defoaming step).

In the mixing step, any appropriate mixing method may be used. There are given, for example, a mixing method using ultrasonic wave irradiation, a mixing method using a centrifugal force, and a mixing method using mechanical stirring.

In the mixing step, two or more different kinds of mixing methods may be used in combination. Mixing by the same mixing method may be repeated twice or more.

In the gelling step, a time period during which the self-assembling peptide aqueous solution is allowed to stand still is generally 1 minute or more, preferably 3 minutes or more, more preferably 5 minutes or more. The temperature at which the self-assembling peptide aqueous solution is allowed to stand still is generally from 4°C to 50°C, preferably from 15°C to 45°C.

In the defoamingstep, any appropriate defoaming method, such as ultrasonic defoaming, vacuum decompression defoaming, or centrifugal defoaming, may be used. Two or more kinds of the defoaming methods may be performed in combination.

The method of manufacturing a visibility-ensuring material described herein may include any appropriate other steps as necessary. As the other steps, there are given a purification step, such as filtration, sterilization steps, such as high-pressure steam sterilization, radiation sterilization, and dry-heat sterilization, a dispensing step into a package container, and the like.

[C. Discharge Device for Visibility-ensuring Material]

The present invention also provides a discharge device for a visibility-ensuring material. The discharge device for a visibility-ensuring material described herein includes a syringe and at least one of: a mesh filter, a needle, and an application nozzle. In one embodiment, the discharge device includes: a syringe including an outer casing and a plunger, the outer casing including an outer casing main body part configured to accommodate a viscoelastic body and a nozzle part arranged in a tip end portion of the outer casing main body part and having an opened tip end, the plunger including an axis part, a gasket mounted at a tip end of the axis part, and a pressure part arranged at a back end of the axis part; and a filter, a needle, an application nozzle, or a spray unit mounted on the outer casing. There is no limitation on a mounting site of the filter as long as the viscoelastic body accommodated in the outer casing main body part passes through the filter to be discharged outside. From the viewpoint of maximizing a discharge amount, the filter is preferably mounted in the vicinity of the nozzle part.

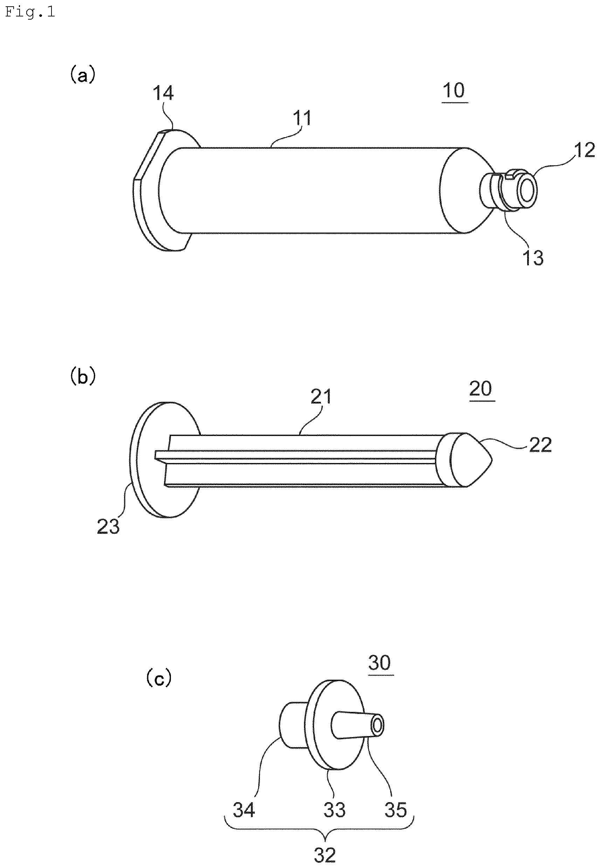

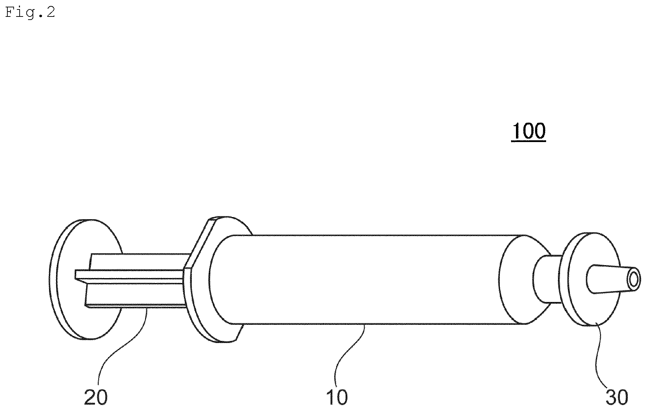

FIGS. **1** are each a schematic perspective view for illustrating an exploded state of a discharge device according to one embodiment of the present invention. FIG. **1(a)** is an illustration of an outer casing, FIG. **1(b)** is an illustration of a plunger, and FIG. **1(c)** is an illustration of a filter unit. FIG. **2** is a schematic perspective view for illustrating an assembled state of the discharge device illustration FIGS. **1.** When description is made with reference to the drawings, the right side of the drawings is defined as a tip end side, and the left side thereof is defined as a back end side. A syringe-type discharge device **100** includes an outer casing **10,** a plunger **20,** and a filter unit **30.**

The outer casing **10** includes a tubular outer casing main body part **11** having an opened back end and a nozzle part **12** arranged in a tip end portion of the outer casing main body part **11** and having an opened tip end. The nozzle part **12** has a tubular shape that is reduced in diameter as compared to the outer casing main body part **11** and is integrally formed with the outer casing main body part. In the illustrated example, a luer lock screw **13** is arranged on an outer periphery of the nozzle part **12**. With this, the nozzle part **12** can be connected to the filter unit **30** through intermediation of a luer lock mechanism. The luer lock screw is not necessarily required, and the nozzle part **12** and the filter unit **30** maybe connected to each other through another system, such as a luer slip system. In the illustrated example, the outer casing **10** further includes a flange **14**. The flange **14** is arranged so as to protrude perpendicularly outward from a back end opening peripheral edge of the outer casing main body part **11.**

As a material for forming the outer casing **10,** any appropriate material may be used depending on the purpose and the like. For example, a resin material, e.g., polyvinyl chloride, polyethylene, polypropylene, polystyrene, polyester, polycarbonate, or polycycloolefin may be preferably used.

The plunger **20** includes an axis part **21,** a gasket **22** mounted at a tip end of the axis part **21,** and a pressure part **23** arranged at a back end of the axis part **21**. The axis part **21** is formed into a bar shape having a section of a circular shape, a cross-like shape, or the like (in the illustrated example, the section has a cross-like shape). The gasket **22** is mounted on the tip end of the axis part **21** through fitting, screwing, adhesion, or the like. The gasket **22** is formed so that its outer peripheral surface is slidably brought into close contact with an inner wall of the outer casing **10** when the plunger **20** is advanced or retracted in the outer casing **10**. The gasket **22** is formed so as to conform to the tip end portion of the outer casing main body part **11** so that a gap is not formed when the gasket **22** is inserted into the tip end of the outer casing main body part **11**. The pressure part **23** has a disc shape and is integrally formed with the axis part. The plunger **20** can be moved toward the tip end of the outer casing **10** by pressing the pressure part **23** with a finger or the like.

As a material for forming the **axis part 21** and the pressure part **23,** any appropriate material may be used depending on the purpose and the like. For example, resin materials, such as polyvinyl chloride, polyethylene, polypropylene, polystyrene, polyester, and polycarbonate, may be preferably used.

As a material for forming the gasket **22,** elastic materials, such as a rubber material, e.g., a natural rubber, an isoprene rubber, a styrene-butadiene rubber, a chlorinated butyl rubber, or a silicone rubber, and a thermoplastic elastomer material, e.g., a urethane-based elastomer, an ester-based elastomer, or a styrene-based elastomer, may be preferably used.

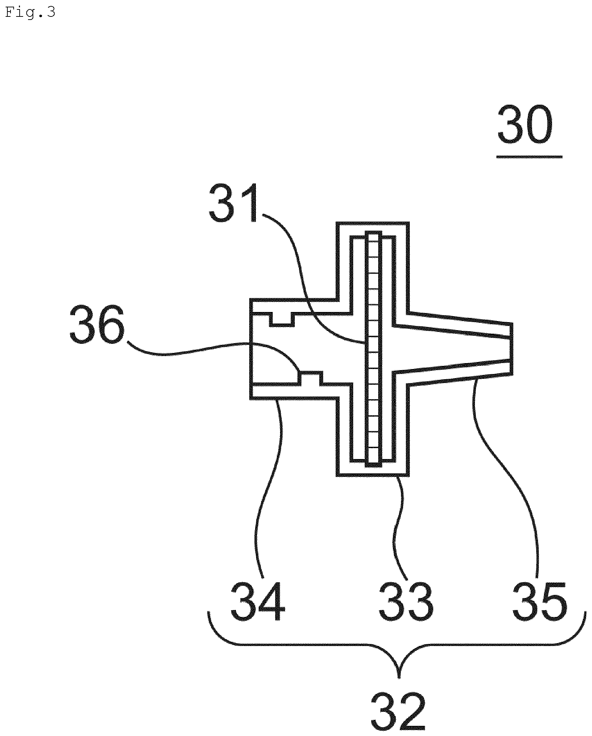

FIG. **3** is a schematic sectional view of the filter unit **30**. The filter unit **30** includes a filter **31** and a housing **32** configured to accommodate the filter **31**. The housing **32** includes a disc-shaped filter accommodating part **33** and a supply port part **34** and an outlet port part **35** which protrude perpendicularly from the filter accommodating part **33**. In this embodiment, the outlet port part **35** corresponds to the discharge port part of the discharge device **100.**

A luer lock screw **36** is arranged on an inner periphery of the supply port part **34,** and with this, the filter unit **30** can be connected to the nozzle part of the outer casing through intermediation of the luer lock mechanism. Unlike the illustrated example, a luer lock screw may also be arranged on an outer periphery or an inner periphery of the outlet port part **35**. In this case, other components, such as a catheter, a needle, and a cap, and the outlet port part **35** can be connected to each other through intermediation of the luer lock mechanism. Alternatively, as a connection system with respect to the supply port part and/or the outlet port part, a luer through system may be adopted.

The filter **31** is as described above.

As the filter, a commercially available filter may be used. Specific examples of the commercially available filter include "NITEX", "PETEX", and"PROPYLTEX" (trade names) manufactured by Sefar AG, and "Nylon Net Filter" (trade name) manufactured by Merck Millipore. As a mesh filter unit, a commercially available mesh filter unit may be used. As a specific example of the commercially available mesh filter unit, there are given a cell suspension filtration item "Falcon S" (trade name) manufactured by BioLab Co., Ltd., and the like.

As the needle, a commercially available needle may be used. Specific examples of the commercially available needle include an ophthalmic needle NIP-015 (30 G×22 mm), an ophthalmic needle (straight) NIP-013 (27 G×22 mm), an ophthalmic needle NIP-014 (25 G×22 mm), and an ophthalmic needle (curved) NIP-012 (27 G×22 mm), all of which are manufactured by Nipro Corporation. The needle is mounted onto the nozzle part through any appropriate means (e.g., luer lock mechanism).

As the application nozzle, a commercially available application nozzle may be used. As a specific example of the commercially available application nozzle, there are given "Double thread screwed tapered nozzle" (product name), gauge 18, manufactured by Musashi Engineering, Inc., and the like. The application nozzle is mounted onto the nozzle part through any appropriate means (e.g., luer lock mechanism).

The spray unit is as described above, and a commercially available spray unit may be used. The spray unit is mounted onto the nozzle part through any appropriate means (e.g., luer lock mechanism).

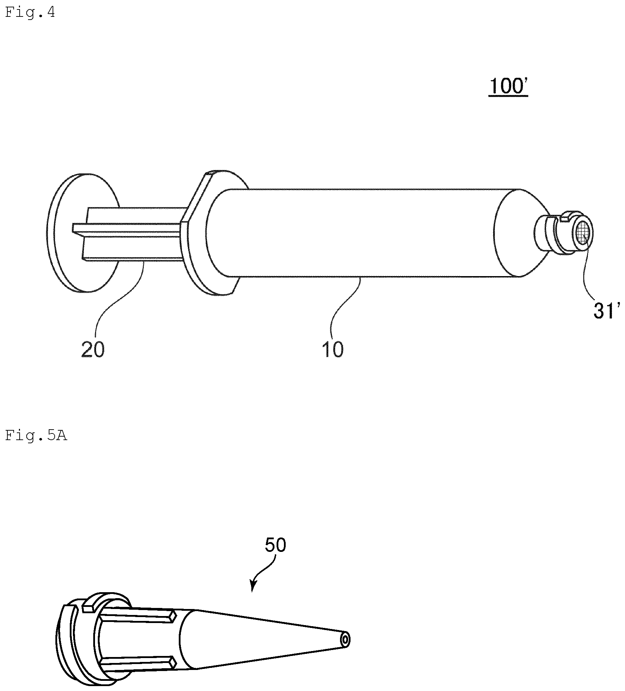

FIG. **4** is a schematic perspective view for illustrating an assembled state of a discharge device according to another embodiment of the present invention. In a discharge device **100'**, a filter **31'** is directly mounted on the outer casing **10.** Specifically, the filter **31'** may be formed on the opening at the tip end of the nozzle part through adhesion, fusion, or integral molding.

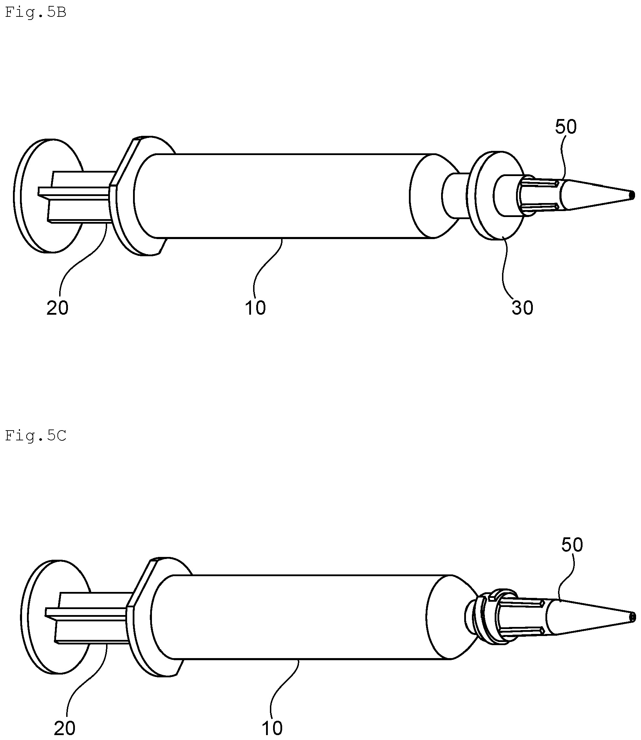

In one embodiment, an application nozzle may be mounted in a tip end portion of the discharge device. The application nozzle may have, for example, a tapered shape as illustrated in FIG. **5A.** Through mounting of such application nozzle, a gel can be properly applied to an affected area, and hence more preferred discharge can be realized. As means for mounting the application nozzle onto the discharge device, any appropriate means may be adopted in accordance with the configuration of a mounting device. For example, the application nozzle of FIG. **5A** includes an external screw as means for mounting onto the discharge device but may include an internal screw. For example, the mode in which the application nozzle is mounted onto the discharge device illustrated in FIG. **2** is illustrated in FIG. **5B,** and the mode in which the application nozzle is mounted onto the discharge device illustrated in FIG. **4** is illustrated in FIG. **5C.**

The discharge device of the present invention may be a prefilled-type discharge device configured to accommodate a gel-like viscoelastic body in advance. Alternatively, a gel-like viscoelastic body may be accommodated (filled) into the discharge device for each use. The gel-like viscoelastic body is accommodated into a space defined by the outer casing, the gasket, and the filter.

The discharge device of the present invention may be subjected to high-pressure steam sterilization (for example, 121°C, 2 atm, 15 minutes). When the discharge device accommodating a visibility-ensuring material is subjected to high-pressure steam sterilization in advance, the discharge device can be used immediately. Thus, the discharge device can be excellent in convenience at a time of use. The discharge device can also be used for emergency.

Examples

Now, the present invention is specifically described on the basis of Examples, but the present invention is not limited by these Examples.

[Method of measuring Storage Elastic Modulus]

A storage elastic modulus G' of a viscoelastic body was measured through use of a rotary rheometer ("Advanced Rheometer AR 1000" (product name) manufactured by TA Instruments Japan Inc.) serving as a dynamic viscoelastic measurement device. Specifically, the measurement was performed as follows. First, a geometry (cone made of aluminum, diameter: 20 mm, cone angle: 1°, gap: 24 μm) to be brought into contact with a sample and a constant-temperature reservoir configured to keep a sample stage at a predetermined temperature were mounted on the rheometer. Then, the measurement was performed by the following measurement procedure under the measurement conditions of a temperature of 37°C, a torque of 1 μN·m, a frequency of from 0.5 radian/second to 100 radians/second, and a value at 1 radian/second was defined as the storage elastic modulus G'.

(1) About 55 μL of a sample is placed on the sample stage of the rheometer.

(2) The geometry is moved to a gap of 24 μm from the sample stage and brought into contact with the sample.

(3) The geometry is slightly moved to be fitted with the sample.

(4) 15 seconds after the movement of the geometry is stopped (during the 15 seconds, a solvent trap for preventing volatilization of a solvent from the sample is placed), the measurement is started.

In the case of using a discharge device, an obtained gel-like viscoelastic body was loaded into an application nozzle ("Double thread screwed tapered nozzle" (product name), gauge 18, manufactured by Musashi Engineering, Inc.), a needle (Code: 00-222, disposable ophthalmic needle, 27 G (0.20 mm) ×22 mm, blunt needle, manufactured by Nipro Corporation), or a syringe having a needle and the following mesh filter housing unit mounted thereon, and ejected into a vial. Then, about 55 µL of the sample was collected from the vial through use of a variable volume pipette and similarly measured for the storage elastic modulus G'.

<Mesh Filter Housing Unit>

Mesh filter: manufactured by Sefar AG, SEFAR PETEX (trademark) (polyethylene terephthalate, opening: 11 µm, filter diameter: φ13 mm (actual use area except the welded portion: φ10 mm)
Housing: made of polypropylene

[Method of measuring Extrusion Force]

An extrusion force of a discharge device was measured through use of a precision universal tester (Autograph AG-IS MS type manufactured by Shimadzu Corporation). Specifically, an extrusion device was fixed with a discharge part placed downward, and a force applied when a plunger was pushed from above at a speed of 100 mm/min was measured.

[Method of measuring Spread Area of Viscoelastic Body]

Viscoelastic bodies of Examples 6 to 15 and Comparative Examples 1 and 2 were each measured for a spread area by the following procedure.

(1) A digital camera (D40X (product name) manufactured by Nikon Corporation, lens: 50 mm DG MACRO manufactured by Sigma Corporation) was mounted on a fixed stage so that the lens faced directly downward.

(2) Plotting paper was placed below the digital camera, and a glass sheet was mounted on the plotting paper.

(3) The above-mentioned mesh filter housing unit and needle were mounted on the tip of a syringe accommodating a test sample as necessary.

(4) The tip end of the needle or the syringe was brought into contact with the glass sheet, and a plunger was pressed while care was taken so that the position of the tip end was not moved. Thus, about 1 mL of the test sample was ejected.

(5) 20 seconds after the ejection, the test sample was photographed with the digital camera. The test was conducted at room temperature. The photograph was printed onto A4 plain paper.

(6) A portion corresponding to 1 cm$^2$ and a portion in which the test sample was spread were respectively cut out, and each weight of the paper was weighed with an electronic scale.

(7) The value of the weight of the paper in the portion in which the test sample was spread was divided by the value of the weight of the paper in the portion corresponding to 1 cm$^2$, to thereby calculate the area of the portion in which the test sample was spread.

[Test of Visibility in Blood]

Viscoelastic bodies of Examples 6 to 16 and Comparative Examples 2 and 3 were each subjected to a visibility test by the following procedure.

(1) Blood of a rabbit (New Zealand White) was collected, and a heparin sodium injection (manufactured by Mochida Pharmaceutical Co., Ltd.) was added to the collected blood.

(2) About 2 mL of the blood containing heparin sodium obtained in (1) was placed into a 35 mm petri dish (Falcon dish).

(3) The above-mentioned needle or the above-mentioned needle and mesh filter housing unit were mounted onto the tip of a syringe accommodating a test sample.

(4) About 0.6 mL of the test sample was injected into the petri dish.

(5) 5 minutes after the injection of the test sample, the state of each sample was photographed with the digital camera.

(6) The case where the bottom of the petri dish was seen clearly was determined to be satisfactory visibility (○), and the case where the bottom was not seen clearly was determined to be unsatisfactory visibility (×).

[Example 1]

A prefilled-type discharge device that was a discharge device as illustrated in FIGS. **1** and FIG. **2** was produced. Specifically, 5 ml of a self-assembling peptide gel ("PanaceaGel" (product name), peptide concentration: 1.5 wt%, manufactured by Menicon Co., Ltd.) was filled into an outer casing of a syringe ("ClearJect" (product name), volume: 5 ml, manufactured by Taisei Kako Co., Ltd.) including the outer casing that included a nozzle part and a fringe and a plunger that included a gasket and a pressure part. Then, the plunger was inserted into the outer casing so that a gap was not formed in the outer casing. After that, a supply port part of a filter unit ("Falcon S" (product name) manufactured by BioLab Co., Ltd., the filter was exchanged by cutting a nylon net filter having an opening of 11 $\mu$m, manufactured by Merck Millipore Corporation) was connected to the nozzle part of the outer casing, to thereby produce a discharge device.
The fringe and the pressure part of the obtained discharge device were held with a finger, and the plunger was pressed into the outer casing, to thereby discharge the self-assembling peptide gel. As a result, the self-assembling peptide gel was able to be discharged easily with a very small extrusion pressure. The discharged gel had appropriate flowability and was excellent in adhesiveness and covering property with respect to a skin. The surface of the gel was smooth and excellent in transparency. When an affected area was confirmed through the gel, blood was pushed away from the affected area, and the state of the affected area was able to be satisfactorily confirmed.

[Example 2]

A discharge device was produced in the same manner as in Example 1 except that "Falcon S" (product name) (opening of the filter: 30 $\mu$m) manufactured by BioLab Co. , Ltd. was used as the filter unit. The self-assembling peptide gel was discharged from the discharge device in the same manner as in Example 1. As a result, the self-assembling peptide gel was able to be discharged easily with a small extrusion pressure. The discharged gel had appropriate flowability and was excellent in adhesiveness and covering property with respect to a skin. The surface of the gel was smooth and excellent in transparency.

[Example 3]

A discharge device was produced in the same manner as in Example 1 except that "Falcon S" (product name) (opening of the filter: 200 $\mu$m) manufactured by BioLab Co., Ltd. was used as the filter unit. The self-assembling peptide gel was discharged from the discharge device in the same manner as in Example 1. As a result, the self-assembling peptide gel was able to be discharged easily with a small extrusion pressure . The discharged gel had appropriate flowability and was excellent in adhesiveness and covering property with respect to a skin. The surface of the gel was smooth and excellent in transparency.

[Example 4]

A prefilled-type discharge device that was a discharge device as illustrated in FIG. **5B** was produced. Specifically, 5 ml of a self-assembling peptide gel ("PanaceaGel" (product name), peptide concentration: 1.5wt%, manufactured by Menicon Co., Ltd.) was filled into an outer casing of a syringe (having a shape illustrated in FIGS. **1** and FIG. **2,** "ClearJect" (product name), volume: 5 ml, manufactured by Taisei Kako Co., Ltd.) including the outer casing that included a nozzle part and a fringe and a plunger that included a gasket and a pressure part. Then, the plunger was inserted into the outer casing so that a gap was not formed in the outer casing. After that, a supply port part of a filter unit (having a shape illustrated in FIG. **3,** the filter being a mesh filter PETEX 07-11/5 having an opening of 11 $\mu$m and an opening ratio of 5%, manufactured by Sefar AG) was connected to the nozzle part of the outer casing. Further, an application nozzle ("Double thread screwed tapered nozzle" (product name), gauge 18, having a shape illustrated in FIG. **5A,** manufactured by Musashi Engineering, Inc.) was connected to an outlet port part of the filter unit, to thereby produce a discharge device. The nozzle part and the supply port part, and the outlet port part and the application nozzle were respectively connected to each other through a luer lock mechanism.
The fringe and the pressure part of the obtained discharge device were held with a finger, and the plunger was pressed into the outer casing, to thereby discharge the self-assembling peptide gel. As a result, the self-assembling peptide gel was able to be discharged easily with a very small extrusion pressure. The extrusion force of the similar discharge device was measured to be 4.2 N. When the discharged gel was applied onto a resected surface of a liver of a guinea pig, the adhesiveness and covering property of the gel with respect to the resected surface were excellent, and blood was satisfactorily pushed away. The surface of the gel was smooth and excellent in transparency, and the state of an affected area was able to be satisfactorily confirmed through the gel.

[Example 5]

A prefilled-type discharge device that was a discharge device as illustrated in FIG. **2,** including a needle (ophthalmic needle (straight) NIP-013 (27 G× 22 mm) manufactured by Nipro Corporation) instead of the filter unit (**30**) was produced. The self-assembling peptide gel was discharged from the discharge device in the same manner as in Example 1. As a result, the discharged gel had appropriate flowability and was excellent in adhesiveness and covering property with respect to a skin. Further, the surface of the gel was smooth and excellent in transparency, and the state of an affected area was able to be satisfactorily confirmed through the gel. Meanwhile, when an attempt was made to apply a large amount of the gel to a wide range, a high extrusion force was required, and the workability of the application to the affected area was slightly degraded. The extrusion force of the similar discharge device was measured to be 44.3 N.

(Comparative Example 1)

A discharge device was produced in the same manner as in Examples 1 to 3 except that the filter unit was not connected to the nozzle part. The self-assembling peptide gel was discharged from the discharge device in the same manner as in Examples 1 to 3.

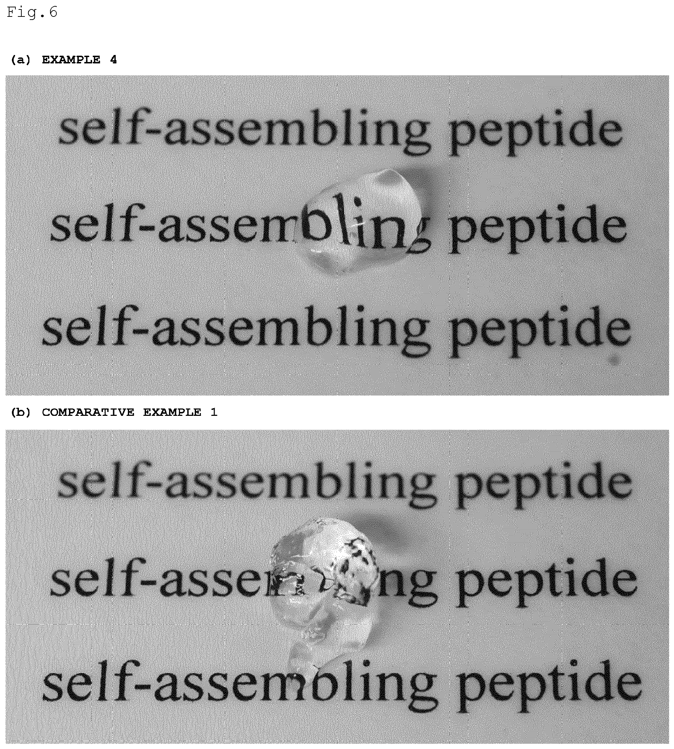

FIG. **6(a)** is a photograph of a self-assembling peptide gel discharged from the discharge device obtained in Example 4, and FIG. **6(b)** is a photograph of a self-assembling peptide gel discharged from the discharge device obtained in Comparative Example 1. As is understood from those photographs, the gel having passed through the filter or the application nozzle has a smooth surface and is excellent in visibility as well as adhesiveness and covering property in the case of being applied to a wound area. The reason for this is considered as follows. The gel passes through a fine filter opening or a thin nozzle tip end portion and then self-assembles again, to thereby form a gel having high uniformity again.

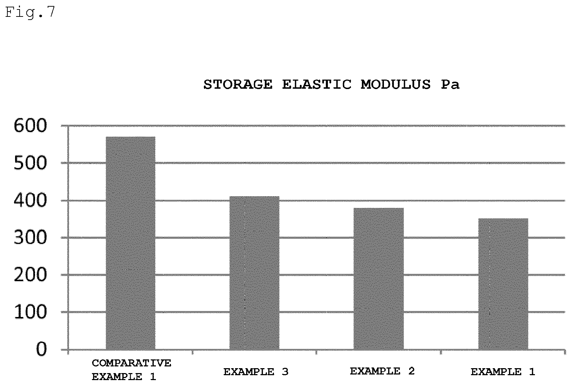

FIG. **7** is a graph for showing storage elastic moduli of self-assembling peptide gels after being discharged from the discharge devices obtained in Examples 1 to 3 and Comparative Example 1. The storage elastic moduli of the gels of Examples 1 to 3 and Comparative Example 1 are 351 (Pa), 381 (Pa), 410 (Pa), and 571 (Pa), respectively. Thus, it is understood that, when the gel is caused to pass through the filter, a decrease ratio of the storage elastic modulus can be improved as compared to the case where the gel is not caused to pass through the filter. Specifically, when the gel is caused to pass through the filter, the storage elastic modulus of the gel is decreased by from about 20% to about 40%.

[Example 6]

A visibility-ensuring material having a composition shown in Table 1 was prepared. The specific procedure is as follows. That is, self-assembling peptide (SPG-178: peptide of SEQ ID NO: 1 having an acetylated N-terminus and an amidated C-terminus) hydrochloride and trehalose dihydrate were weighed into a container. Then, water was supplied to the container, and the container was covered with a lid. The container was set in a planetary centrifugal mixer ("ARE-310" (Product No.) manufactured by Thinky Corporation) using a container having an irregular shape, and the resultant was stirred. L-Histidine was added to the obtained mixed solution to adjust a pH, and then the mixture was stirred with the planetary centrifugal mixer. The obtained solution was defoamed and deaerated with the planetary centrifugal mixer, to thereby provide a self-assembling peptide aqueous solution.

The obtained self-assembling peptide aqueous solution was dispensed into a syringe by 5 g. Then, the syringe was capped, and a plunger was mounted thereon. The resultant was placed in a sterilization bag and subjected to autoclave treatment at 121°C for 20 minutes. The self-assembling peptide aqueous solution after being subjected to the autoclave treatment was naturally cooled to room temperature, to thereby provide a visibility-ensuring material. The obtained visibility-ensuring material discharged from the following discharge device was evaluated for a storage elastic modulus, spreading of the obtained visibility-ensuring material after being applied, and visibility in blood. The results are shown in Table 1.

<Discharge Device>

Needle (manufactured by Nipro Corporation, Code: 00-222, disposable ophthalmic needle, 27 G (0.20 mm)×22 mm, blunt needle)

Mesh filter housing unit: mesh filter (manufactured by Sefar AG, SEFAR PETEX (trademark) (polyethylene terephthalate, opening: 11 $\mu$m, filter diameter: $\varphi$13mm (actual use area except the welded portion: $\varphi$10 mm)), housing: made of polypropylene

Syringe: manufactured by Taisei Kako Co., Ltd., product name "ClearJect", volume: 5 ml

[Example 7]

A visibility-ensuring material having a composition shown in Table 1 was prepared. The specific procedure is as follows. That is, self-assembling peptide (SPG-178) hydrochloride and trehalose dihydrate were weighed and loaded into a centrifuge tube. Then, water was supplied to the centrifuge tube, and the resultant was treated to be mixed at an output of 500 W for 5 minutes through use of an ultrasonic homogenizer ("Vibra-Cell VC-505" (Product No.) manufactured by Sonic & Material, Inc.). The obtained mixed solution was defoamed by centrifugation at 3,000 rpm for 5 minutes and further deaerated under reduced pressure through use of an aspirator. After the deaeration, a 50 mM $Na_2CO_3$ aqueous solution was added to the mixed solution to adjust a pH, and then, the resultant was treated to be mixed at an output of 500 W for 5 minutes through use of the ultrasonic homogenizer. The mixed solution was deaerated under reduced pressure through use of the aspirator, to thereby provide a self-assembling peptide aqueous solution.

[Table 1]

| | Viscoelastic body | Additive | Concentration of additive (w/w%) | pH adjuster | pH | Gel production method | Presence or absence of discharge device | Storage elastic modulus (Pa)*1 | Spread area (cm²) | Visibility |
|---|---|---|---|---|---|---|---|---|---|---|
| Example 6 | Self-assembling peptide gel (Peptide: SPG-178 hydrochloride) (Peptide concentration: 1.5 w/w%) | Trehalose hydrate | 7.2 | L-Histidine | 5.9 | Planetary centrifugal mixer using container having irregular shape | Present | 288 | 3.5 | ○ |
| Example 7 | Self-assembling peptide gel (Peptide: SPG-178 hydrochloride) (Peptide concentration: 2.0 w/w%) | Trehalose hydrate | 8.5 | Sodium carbonate | 5.9 | Ultrasonic homogenizer | Present | 661 | 2.2 | ○ |
| Example 8 | Self-assembling peptide gel (Peptide: SPG-178 hydrochloride) (Peptide concentration: 1.5 w/w%) | - | - | Sodium carbonate | 6.8 | Planetary centrifugal mixer using container having irregular shape | Present | 321 | 3.3 | ○ |
| Example 9 | Self-assembling peptide gel (Peptide: SPG-221 hydrochloride) (Peptide concentration: 1.5 w/w%) | Trehalose hydrate | 7.9 | Sodium carbonate | 7.9 | Planetary centrifugal mixer using container having irregular shape | Present | 168 | 3.0 | ○ |
| Example 10 | Self-assembling peptide gel (Peptide: SPG-178 hydrochloride) (Peptide concentration: 1.5 w/w%) | Trehalose hydrate | 7.9 | Sodium carbonate | 6.5 | Planetary centrifugal mixer using container having irregular shape | Present | 260 | 3.3 | ○ |
| Example 11 | Self-assembling peptide gel (Peptide: SPG-178 hydrochloride) (Peptide concentration: 0.8 w/w%) | Trehalose hydrate | 8.5 | Sodium carbonate | 6.8 | Planetary centrifugal mixer using container having irregular shape | Present | 42 | 4.4 | ○ |

(continued)

| | Viscoelastic body | Additive | Concentration of additive (w/w%) | pH adjuster | pH | Gel production method | Presence or absence of discharge device | Storage elastic modulus (Pa)[*1] | Spread area (cm²) | Visibility |
|---|---|---|---|---|---|---|---|---|---|---|
| Example 12 | Self-assembling peptide gel (Peptide: SPG-220 hydrochloride) (Peptide concentration: 1.5 w/w%) | Trehalose hydrate | 7. 9 | Sodium carbonate | 7.4 | Planetary centrifugal mixer using container having irregular shape | Present | 645 | 2.5 | ○ |
| Example 13 | Self-assembling peptide gel (Peptide: SPG-220 hydrochloride) (Peptide concentration: 1.2 w/w%) | Trehalose hydrate | 6.32 | Sodium carbonate | 7.2 | Ultrasonic homogenizer | Present | 328 | 2.0 | ○ |
| Example 14 | Self-assembling peptide gel (Peptide: SPG-178 methanesulfonate) (Peptide concentration: 1.5 w/w%) | Sucrose | 10 | Sodium carbonate | 6.1 | Ultrasonic homogenizer | Present | 502 | 2.7 | ○ |
| Example 15 | Polymer gel (Polymer: polyacrylic acid-based polymer) (Polymer concentration: 0.25 w/w%) | - | - | - | 8.0 | Planetary centrifugal mixer using container without irregularities | Present | 340 | 2.1 | ○ |
| Example 16 | Self-assembling peptide gel (Peptide: SPG-178 hydrochloride) (Peptide concentration: 0.8 w/w%) | Trehalose hydrate | 8.5 | Sodium carbonate | 6.8 | Planetary centrifugal mixer using container having irregular shape | Present | 63 | 3.4 | ○ |
| Comparative Example 2 | Self-assembling peptide gel (Peptide: SPG-178 hydrochloride) (Peptide concentration: 2.0 w/w%) | Trehalose hydrate | 8.5 | Sodium carbonate | 5. 9 | Ultrasonic homogenizer | Absent | 877 | 1.8 | × |
| Comparative Example 3 | Self-assembling peptide gel (Peptide: SPG-178 hydrochloride) (Peptide concentration: 0.4 w/w%) | Trehalose hydrate | 8.5 | Sodium carbonate | 7.8 | Ultrasonic homogenizer | Present | 7 | 5.7 | × |

The obtained self-assembling peptide aqueous solution was dispensed into a syringe by 5 g. Then, the syringe was capped, and a plunger was mounted thereon. The resultant was placed in a sterilization bag and subjected to autoclave treatment at 121°C for 20 minutes. The self-assembling peptide aqueous solution after being subjected to the autoclave treatment was naturally cooled to room temperature, to thereby provide a visibility-ensuring material. The obtained visibility-ensuring material discharged from the following discharge device was evaluated for a storage elastic modulus, spreading of the visibility-ensuring material after being applied, and visibility in blood. The results are shown in Table 1.

<Discharge Device>

Needle (manufactured by Nipro Corporation, Code: 00-222, disposable ophthalmic needle, 27 G (0.20 mm)×22 mm, blunt needle)

Mesh filter housing unit: mesh filter (manufactured by Sefar AG, SEFAR PETEX (trademark) (polyethylene tereph- thalate, opening: 11 $\mu$m, filter diameter : $\phi$13mm (actual use area except the welded portion: $\phi$10 mm))), housing: made of polypropylene

Syringe: manufactured by Taisei Kako Co., Ltd., product name "ClearJect", volume: 5 ml

[Example 8]

A visibility-ensuring material having a composition shown in Table 1 was obtained in the same manner as in Example 6 except that the trehalose dihydrate was not used, and a 100 mM $Na_2CO_3$ aqueous solution was used instead of the L-histidine to adjust a pH. The obtained visibility-ensuring material discharged from the discharge device used in Example 6 was evaluated for a storage elastic modulus, spreading of the visibility-ensuring material after being applied, and visibility in blood. The results are shown in Table 1.

[Example 9]

A visibility-ensuring material having a composition shown in Table 1 was obtained in the same manner as in Example 6 except that SPG-221 (peptide of SEQ ID NO: 19 having an acetylated N-terminus and an amidated C-terminus) was used as the self-assembling peptide, and a 100 mM$Na_2CO_3$ aqueous solution was used instead of the L-histidine to adjust a pH. The obtained visibility-ensuring material discharged from the discharge device used in Example 6 was evaluated for a storage elastic modulus, spreading of the visibility-ensuring material after being applied, and visibility in blood. The results are shown in Table 1.

(Comparative Example 2)

A visibility-ensuring material having a composition shown in Table 1 was prepared in the same manner as in Example 7. The obtained visibility-ensuring material was evaluated for a storage elastic modulus, spreading after being applied, and visibility in blood. The results are shown in Table 1.

(Comparative Example 3)

A visibility-ensuring material having a composition shown in Table 1 was prepared in the same manner as in Example 7 except that the concentration of the self-assembling peptide was set to 0.4 w/w%. The obtained visibility-ensuring material discharged from the discharge device used in Example 6 was evaluated for a storage elastic modulus, spreading of the visibility-ensuring material after being applied, and visibility in blood. The results are shown in Table 1.

[Example 10]

A visibility-ensuring material having a composition shown in Table 1 was obtained in the same manner as in Example 6 except that the addition amount of the trehalose dihydrate was changed, and a 100 mM $Na_2CO_3$ aqueous solution was used instead of the L-histidine to adjust the pH. The obtained visibility-ensuring material discharged from the following discharge device was tested for a storage elastic modulus, a spread area, and visibility in blood. The results are shown in Table 1.

<Discharge Device>

Needle (manufactured by Nipro Corporation, Code: 00-222, disposable ophthalmic needle, 27 G (0.20 mm) ×22 mm, blunt needle)
Mesh filter housing unit: mesh filter (manufactured by Sefar AG, SEFAR PETEX (trademark) (polyethylene terephthalate, opening: 11 μm, filter diameter : φ13mm (actual use are a except the welded portion: φ10 mm))), housing: made of polypropylene
Syringe: manufactured by Taisei Kako Co., Ltd., product name "ClearJect", volume: 5 ml

[Example 11]

A visibility-ensuring material having a composition shown in Table 1 was prepared in the same manner as in Example 10 except that the addition amount of the trehalose dihydrate was changed, and the concentration of the self-assembling peptide (SPG-178) hydrochloride was setto0.8 w/w%. The obtained visibility-ensuring material discharged from the discharge device used in Example 10 was evaluated for a storage elastic modulus, spreading after being applied, and visibility in blood. The results are shown in Table 1.

[Example 12]

A visibility-ensuring material having a composition shown in Table 1 was prepared in the same manner as in Example 9 except that SPG-220 (peptide of SEQ ID NO: 18 having an acetylated N-terminus and an amidated C-terminus) hydrochloride was used as the self-assembling peptide. The obtained visibility-ensuring material discharged from the discharge device used in Example 9 was evaluated for a storage elastic modulus, spreading after being applied, and visibility in blood. The results are shown in Table 1.

[Example 13]

The sample prepared in Example 12 was loaded into a vial. Then, water was loaded into the vial so that the concentration of the self-assembling peptide SPG-220 hydrochloride was 1.2 w/w%. The resultant was treated to be mixed at an output of 500 W for 30 seconds through use of an ultrasonic homogenizer ("Vibra-Cell VC-505" (Product No.) manufactured by Sonic & Material, Inc.). The obtained mixed solution was defoamed by centrifugation at 1,000 rpm for 5 minutes, to thereby provide a self-assembling peptide aqueous solution.
The obtained self-assembling peptide aqueous solution was dispensed into a syringe. Then, the syringe was capped, and a plunger was mounted thereon, to thereby provide a visibility-ensuring material. The obtained visibility-ensuring material discharged from the following discharge device was evaluated for a storage elastic modulus, spreading after being applied, and visibility in blood. The results are shown in Table 1.

[Example 14]

A visibility-ensuring material having a composition shown in Table 1 was prepared. The specific procedure is as follows. That is, self-assembling peptide (SPG-178) methanesulfonate and sucrose were weighed and loaded into a centrifuge tube. Then, water was supplied to the centrifuge tube, and the resultant was treated to be mixed at an output of 500 W for 5 minutes through use of an ultrasonic homogenizer("Vibra-CellVC-505"(ProductNo.)manufactured by Sonic & Material, Inc.). The obtained mixed solution was defoamed by centrifugation at 3,000 rpm for 5 minutes and further deaerated under reduced pressure through use of an aspirator. After the deaeration, a 50 mM Na$_2$CO$_3$ aqueous solution was added to the mixed solution to adjust a pH, and then, the resultant was treated to be mixed at an output of 500 W for 5 minutes through use of the ultrasonic homogenizer. The mixed solution was deaerated under reduced pressure through use of the aspirator, to thereby provide a self-assembling peptide aqueous solution.
The obtained self-assembling peptide aqueous solution was dispensed into a syringe by 5 g. Then, the syringe was capped, and a plunger was mounted thereon. The resultant was placed in a sterilization bag and subjected to autoclave treatment at 121°C for 20 minutes. The self-assembling peptide aqueous solution after being subjected to the autoclave treatment was naturally cooled to room temperature, to thereby provide a visibility-ensuring material. The obtained visibility-ensuring material discharged from the following discharge device was evaluated for a storage elastic modulus, spreading after being applied, and visibility in blood. The results are shown in Table 1.

<Discharge Device>

Needle (manufactured by Nipro Corporation, Code: 00-222, disposable ophthalmic needle, 27 G (0.20 mm) ×22 mm, blunt needle)
Mesh filter housing unit: mesh filter (manufactured by Sefar AG, SEFAR PETEX (trademark) (polyethylene terephthalate, opening: 11 μm, filterdiameter: φ13mm (actual use area except the welded portion: φ10 mm))), housing: made of polypropylene
Syringe: manufactured by Taisei Kako Co., Ltd., product name "ClearJect", volume: 5 ml

[Example 15]

A visibility-ensuring material having a composition shown in Table 1 was prepared. The specific procedure is as follows. That is, a polyacrylic acid salt ("Rheogic 270" (trade name) manufactured by Toagosei Co., Ltd.) was weighed into a container. Then, water was supplied to the container, and the container was covered with a lid. The container was set in a planetary centrifugal mixer ("ARE-310" (Product No.) manufactured by Thinky Corporation) using a container without irregularities, and the resultant was stirred. The obtained solution was defoamed with the planetary centrifugal mixer, to thereby provide an acrylic polymer aqueous solution.

The obtained acrylic polymer aqueous solution was dispensed into a syringe by 5 g, to thereby provide a visibility-ensuring material. The obtained visibility-ensuring material discharged from the following discharge device was evaluated for a storage elastic modulus, spreading after being applied, and visibility in blood. The results are shown in Table 1.

<Discharge Device>

Needle (manufactured by Nipro Corporation, Code: 00-222, disposable ophthalmic needle, 27 G (0.20 mm) ×22 mm, blunt needle)
Syringe: manufactured by Taisei Kako Co., Ltd., product name "ClearJect", volume: 5 ml

[Example 16]

The visibility-ensuring material discharged from the discharge device in the same manner as in Example 11 except that the following discharge device was used as the discharge device was evaluated for a storage elastic modulus, spreading after being applied, and visibility in blood. The results are shown in Table 1.

<Discharge Device>

Application nozzle: manufactured by Musashi Engineering, Inc., product name "Double thread screwed tapered nozzle", gauge 18
Syringe: manufactured by Taisei Kako Co., Ltd., product name "ClearJect", volume: 5 ml

[Evaluation]

The visibility-ensuring materials of Examples 6 to 16 each had a spread area of 2 cm$^2$ or more, rapidly spread after being applied, and had satisfactory covering property. Even when the visibility-ensuring materials of Examples 6 to 16 were allowed to stand in blood, the bottom surface of a petri dish was able to be satisfactorily confirmed. In the visibility-ensuring materials obtained in Examples 6 and 10, satisfactory visibility was ensured even after 24 hours. Meanwhile, the visibility-ensuring material of Comparative Example 2 was excellent in visibility in blood but had a small spread area. Thus, the visibility-ensuring material of Comparative Example 2 was susceptible to improvement. The visibility-ensuring material of Comparative Example 3 rapidly spread and was also excellent in visibility. However, the visibility-ensuring material of Comparative Example 3 had a small storage elastic modulus and hence was mixed with blood, with the result that it was difficult to ensure visibility in blood.

[Test Example 1]

The visibility-ensuring material of Example 6 was used for trabeculectomy of an eye of a rabbit. When the visibility-ensuring material was discharged onto a dissected part through use of the above-mentioned discharge device, bleeding from a wound surface was pushed away, and the dissected part and a surrounding area thereof were able to be easily observed.

[Test Example 2]

When the visibility-ensuring material of Example 6 was applied onto a resected surface of a liver of a rabbit through use of the above-mentioned discharge device, bleeding from a wound surface was pushed away, and the resected surface was able to be easily observed.

[Test Example 3]

When the visibility-ensuring material of Example 9 was applied onto a resected surface of a liver of a guinea pig through use of the above-mentioned discharge device, bleeding from a wound surface was pushed away, and the resected surface was able to be easily observed.

Industrial Applicability

The visibility-ensuring material described herein may be preferably used for ensuring the visibility in a surgical field during surgery or the like.

Reference Signs List

| | |
|---|---|
| **100** | discharge device |
| **100'** | discharge device |
| **10** | outer casing |
| **11** | outer casing main body part |
| **12** | nozzle part |
| **20** | plunger |
| **22** | gasket |
| **30** | filter unit |
| **31, 31'** | filter |
| **34** | supply port part |
| **35** | outlet port part |

## Claims

1. A discharge device for a visibility-ensuring material comprising:

   a syringe, wherein a gel-like viscoelastic body is filled into the syringe; and
   at least one of: a mesh filter having an opening of less than 1 mm, a needle having an inner diameter of less than 1 mm, and an application nozzle having a tip end having an inner diameter of less than 1 mm,

   wherein the gel-like viscoelastic body comprises a self-assembling peptide, and wherein the gel-like viscoelastic body is intended to be passed through the mesh filter, the needle and/or the application nozzle.

2. The discharge device for a visibility-ensuring material according to claim 1, wherein the mesh filter has a mesh size of more than 10-mesh.

3. The discharge device for a visibility-ensuring material according to claim 1 or claim 2, wherein the sum of charges of amino acid residues constituting the self-assembling peptide at pH 7.0 is from -3 to -1 or from +1 to +3.

4. The discharge device for a visibility-ensuring material according to claim 3, wherein the self-assembling peptide comprises a peptide formed of an amino acid sequence selected from SEQ ID Nos: 1 to 19.

## Patentansprüche

1. Abgabevorrichtung für ein Material zur Sicherstellung der Sichtbarkeit, umfassend:

   eine Spritze, wobei ein gelartiger, viskoelastischer Körper in die Spritze gefüllt ist; und
   wenigstens eines aus: einem Maschenfilter, welcher eine Öffnung von weniger als 1 mm aufweist, einer Nadel, welche einen Innendurchmesser von weniger als 1 mm aufweist, und einer Applikationsdüse, welche ein Spitzenende aufweist, welches einen Innendurchmesser von weniger als 1 mm aufweist,
   wobei der gelartige, viskoelastische Körper ein selbstanordnendes Peptid umfasst und
   wobei der gelartige, viskoelastische Körper dafür vorgesehen ist, durch den Maschenfilter, die Nadel und/oder die Applikationsdüse hindurchgeführt.

2. Abgabevorrichtung für ein Material zur Sicherstellung der Sichtbarkeit nach Anspruch 1, wobei der Maschenfilter eine Maschenweite von mehr als 10 Mesh aufweist.

3. Abgabevorrichtung für ein Material zur Sicherstellung der Sichtbarkeit nach Anspruch 1 oder 2, wobei die Summe der Ladungen von Aminosäureresten, welche das selbstanordnende Peptid ausmachen, bei einem pH-Wert von 7,0 von -3 bis -1 oder von +1 bis +3 beträgt.

4. Abgabevorrichtung für ein Material zur Sicherstellung der Sichtbarkeit nach Anspruch 3, wobei das selbstanordnende Peptid ein Peptid umfasst, welches aus einer Aminosäuresequenz gebildet ist, welche aus den SEQ-ID Nummern: 1 bis 19 ausgewählt ist.

**Revendications**

1. Dispositif de décharge pour un matériau assurant la visibilité comprenant :

   une seringue, dans lequel un corps viscoélastique de type gel est chargé dans la seringue ; et
   au moins un parmi : un filtre à tamis ayant une ouverture inférieure à 1 mm, une aiguille ayant un diamètre interne inférieur à 1 mm, et une buse d'application ayant une extrémité de pointe ayant un diamètre interne inférieur à 1 mm,
   dans lequel le corps viscoélastique de type gel comprend un peptide d'auto-assemblage,
   et dans lequel le corps viscoélastique de type gel est destiné à être amené à passer à travers le filtre à tamis, l'aiguille et/ou la buse d'application.

2. Dispositif de décharge pour un matériau assurant la visibilité selon la revendication 1, dans lequel le filtre à tamis a une taille de maille supérieure à 10 mesh.

3. Dispositif de décharge pour un matériau assurant la visibilité selon la revendication 1 ou la revendication 2, dans lequel la somme de charges de résidus d'acide aminé constituant le peptide d'auto-assemblage à un pH 7,0 est de -3 à -1 ou de +1 à +3.

4. Dispositif de décharge pour un matériau assurant la visibilité selon la revendication 3, dans lequel le peptide d'auto-assemblage comprend un peptide formé à partir d'une séquence d'acides aminés choisie parmi SEQ ID NO : 1 à 19.

Fig.1

(a)

14  11  10

12

13

(b)

21  20

22

23

(c)  30

34  33  35

32

Fig.2

<u>100</u>

20                    10                    30

Fig.3

Fig.4

<u>100'</u>

31'

20                     10

Fig.5A

50

Fig.5B

Fig.5C

Fig.6

(a) EXAMPLE 4

(b) COMPARATIVE EXAMPLE 1

Fig.7

**STORAGE ELASTIC MODULUS Pa**

## REFERENCES CITED IN THE DESCRIPTION

**Patent documents cited in the description**

- WO 9917821 A
- US 5670483 A
- WO 2010103887 A1
- JP 5204646 B
- WO 2006116524 A1
- WO 2007000979 A1
- JP 2014143044 A